# EUROPEAN PATENT APPLICATION

(11) **EP 2 238 902 A1**
(43) Date of publication of application: **13.10.2010**
(21) Application number: 10158007.4
(22) Date of filing: 26.03.2010
(51) Int. Cl.: A61B 5/08, A61B 5/1455, A61B 5/087, G01K 13/00, A61B 5/0205, A61B 5/00

(54) **Ear wearable monitoring system**

(30) Priority: 03.04.2009 US 166374 P; 29.05.2009 US 474690
(71) Applicant: GENERAL ELECTRIC COMPANY, Schenectady, NY 12345 (US)
(72) Inventor: Haveri, Heikki, 03150, Humari (FI); Leppala, Kristina, 01600, Vantaa (FI)
(74) Representative: Illingworth-Law, William Illingworth

(57) **Abstract**

A wearable monitoring system to collect medical information from a subject is disclosed herein. The wearable monitoring system includes a housing (5) having electronics (6) for managing a signal and which at least one housing is operatively connectable to at least one sensor (10,20) for transmitting to the housing (5) the signal acquired by the at least one sensor. The electronics (6) of the housing (5) is for managing the signal which is indicative of a condition of a respiration function and which housing is installable to the subject's ear (2) or its vicinity.

## Description

### BACKGROUND OF THE INVENTION

This disclosure relates generally to a wearable monitoring system to collect medical information from a subject.

During apnea there is no movement of muscles of a respiration and a volume of lungs initially remains unchanged. Depending on an openness of airways there may not be a flow of a breathing gas between the lungs and an ambient during apnea. Apnea can be drug-induced (e.g., opiate toxicity), mechanically induced (e.g., strangulation or choking), or it can occur as a consequence of neurological disease or trauma as was explained above.

Many people in hospital wards suffer from apnea, caused by opiates or other medicine. Patients may also have obstructive apnea, which is caused by a blockage in the airways, such as a thong or similar physical obstruction. Patients usually don't have any monitoring connected on as it is rather expensive and there are usually many patients in the ward. Furthermore, there is also a shortage of nursing personnel keeping an eye on patients continuously.

Many healthy people suffer from sleep apnea that can be divided into three distinct forms: central, obstructive, and a complex sleep apnea, which is a combination of central and obstructive apnea. In central sleep apnea, breathing is interrupted by the lack of respiratory effort. In obstructive sleep apnea, breathing is interrupted by a physical block to the airflow despite the respiratory effort. In complex sleep apnea, there is a transition from central to obstructive features during the events themselves. Central apnea is commonly caused by neurological etc. characteristics, whereas obstructive apnea is caused by for example over weight.

Many elderly people at home have to take a lot of different medical pills or other type of medicine, which may cause apnea. Also many infants, small babies and even small children up to three years age suffer from sudden infant death syndrome (SIDS). It is also very stress full for many parents, as they can not sleep well during nights, since they compulsively wake up to see how their small babies and children are sleeping.

As the respiratory airflow measurement can be used for measuring the respiration rate and apnea, it is also important to know about the patient's systemic changes. Pulse oximetry is a non-invasive method allowing the monitoring of the oxygenation of a patient's hemoglobin. A sensor is placed on a thin part of the patient's anatomy, usually a fingertip or earlobe, or in the case of a neonate, across a foot, and a light containing both red and infrared wavelengths is passed from one side to the other. Changing absorbance of each of the two wavelengths is measured, allowing determination of the absorbances due to the pulsing arteria blood alone, excluding venous blood, skin, bone, muscle and fat. Based upon the ratio of changing absorbance of the red and infrared light caused by the difference in color between oxygen-bound (bright red) and oxygen unbound (dark red or blue, in severe cases) blood hemoglobin, a measure of oxygenation (the per cent of hemoglobin molecules bound with oxygen molecules) can be made.

Pulse oximetry data is necessary whenever a patient's oxygenation is unstable, including intensive care, critical care, and emergency department areas of a hospital. Data can also be obtained for the assessment of any patient's oxygenation in primary care. Although pulse oximetry is used to monitor oxygenation, it cannot determine the metabolism of oxygen, or the amount of oxygen being used by a patient. However, the use of pulse oximetry to detect hypoventilation is impaired with the use of supplemental oxygen, as it is only when patients breathe room air that abnormalities in respiratory function can be detected reliably with its use. Therefore, the routine administration of supplemental oxygen may be unwarranted if the patient is able to maintain adequate oxygenation in room air, since it can result in hypoventilation going undetected.

Most devices, such as impedance measurement, resistive belts, or piezo-resistive belts etc., do not measure the actual flow of the breathing gas through the nose or the mouth, but they measure for example the respiratory muscle movement or chest movement. These indirect measurements are unreliable since the respiratory muscle or chest movement may occur even when the patient is suffering from apnea. Devices are also sensitive to motion artefacts and other disturbances. Existing devices usually contain cables that connect the measuring devices to a host, which is used to calculate and show respiration and pulse oximetry waveforms and values. Usually the host also delivers electrical power for the devices.

In many cases a long-term stability and status of a patient needs to be monitored continuously inside the hospitals, but also outside the hospital at home care or, for example in elderly care house. Devices that are placed on the patient's face and contain a cable going over the face, tickle and irritate the patient who then easily sweeps off and disconnects the device from the face. A pulse oximetry containing a cable, which is connected to a fingertip or similar place, disturbs the usage of hands etc. Especially older people tend to rip off everything from their hands unconsciously. Many people, in hospital wards and at home care or elderly care, need to move around the living area and for that reason the device should be wireless, portable, unnoticeable and easy to wear. A device with a cable would disturb everyday life and is not practical.

Disposability is also one of the clinical requirements for the device that is in close contact to patient, especially the patient's airways, since existing cleaning practices are not reliable enough to ensure high enough level of purity for reusable devices. Contaminated reusable devices easily cause a risk of cross contamination between different patients, but non-disposable devices also cause a personal hygiene risk, if they are not cleaned frequently enough, for the patients who already have a lowered level of immunity against bacteria and viruses. Existing devices are usually complex, also containing cables etc., rather expensive, not easy to clean and thus reusable usually.

### BRIEF DESCRIPTION OF THE INVENTION

The above-mentioned shortcomings, disadvantages and problems are addressed herein which will be understood by reading and understanding the following specification.

In an embodiment, a wearable monitoring system to collect medical information from a subject includes a housing having electronics for managing a signal and which at least one housing is operatively connectable to at least one sensor for transmitting to the housing the signal acquired by the at least one sensor. The electronics of the housing is for managing the signal which is indicative of a condition of a respiration function and which housing is installable to the subject's ear or its vicinity.

In another embodiment, a wearable monitoring system to collect medical information from a subject includes a housing having electronics for managing a signal and a cable operatively connectable to at least one sensor for transmitting to the housing the signal acquired by the at least one sensor. The electronics of the housing is for managing the signal acquired along the cable from the at least one sensor and which signal is indicative of a condition of a respiration function and which housing is installable behind an ear of the subject.

In yet another embodiment, a wearable monitoring system to collect medical information from a subject includes at least one sensor for acquiring a signal indicative of a condition of a respiration function and a cable for transmitting the signal acquired by the at least one sensor. The wearable monitoring system to collect medical information from a subject also includes a housing having electronics for managing the signal received along the cable from the at least one sensor and which housing is installable behind an ear of the subject.

Various other features, objects, and advantages of the invention will be made apparent to those skilled in art from the accompanying drawings and detailed description thereof.

### BRIEF DESCRIPTION OF THE DRAWINGS

Figure 1 is a side view of a wearable monitoring system installable to a subject; and
Figure 2 is a side view of a second embodiment of a wearable monitoring system installable to a subject.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

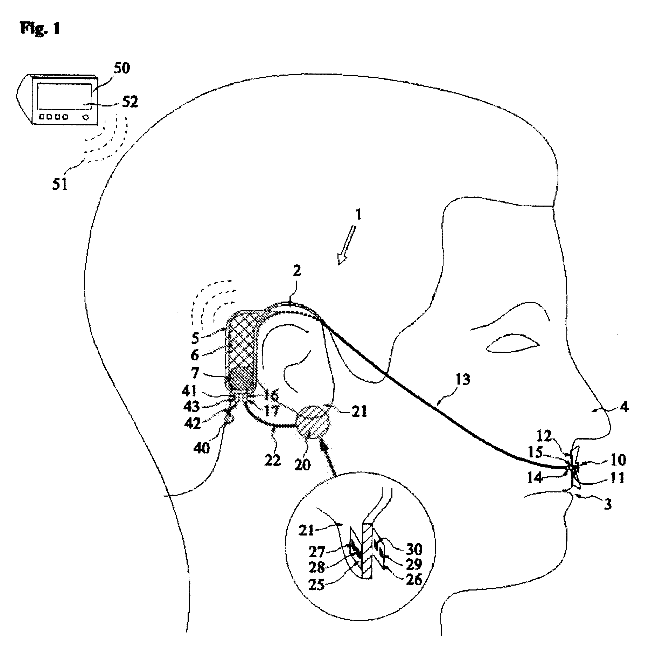

Fig. 1 shows a schematic side view of a wearable monitoring system 1 that may be wireless and can be used to collect a medical information from a subject. The monitoring system 1 can be placed on a side of a subject's head, over an ear 2, extending towards and close to a mouth 3 and a nose 4. The monitoring system 1 comprises a housing 5, which is installable to the subject's ear 2 or its vicinity. A suitable place for the housing is behind the subject's ear 2, similarly as commonly known hearing aid devices are placed. The housing 5 comprises electonics 6 for managing a signal acquired by at least one sensor 10, 20. The electronics 6 may comprise a radio frequency transceiver or similar wireless communication and signal processing. The housing 5 may further comprise a power supply 7, such as a small battery to energize the monitoring system 1. For practical reasons it is advantageous to minimize the size, weight and the power consumption of the monitoring system to keep the size of the housing 5 small, so that it is unnoticeable for the subject wearing the monitoring system 1.

The housing 5 is operatively connectable to the at least one sensor 10, 20, which may be disposable or reusable, for transmitting the signal from the at least one sensor 10, 20 to the housing 5. The signal acquired by the at least one sensor 10, 20 is indicative of a condition of a respiration function. The sensor 10, which is placed between the subject's mouth and nose, is preferably disposable and used for acquiring from the respiration flow through the mouth and/or the nose a signal indicative of at least one of a respiratory flow, respiration rate, one or more gas component and a concentration of a gas component. One of the sensors operatively connectable to the housing 5 can be an ear sensor 20 placed for example on the subject's ear lope 21 or a place such as an auditory canal, which is used for measuring at least one of the oxygen saturation and the pulse rate from the ear.

The sensor 10, for acquiring the respiratory flow signal and/or respiration rate signal, is substantially insensitive to any motion or mechanical vibrations and can be used to acquire the signal through the nose, the mouth or the both at the same time and can also detect apnea. The measurement can be based on measuring a thermal component of the respiration gas flow with at least one respiration detector 11 such as a thermistor. Thermal changes of the respiration gas flowing by the respiration detector 11 change the thermistor's resistance, which is converted into a continuous electrical signal. The amplitude of the signal is proportional to the flow rate of the respiration gas and the frequency is proportional to the respiration rate (RR). These signals can also be used for detecting apnea. Also it is possible to use the respiration detector 11 to acquire a signal indicative of one or more gas component or their concentration in the respiration gas flow.

The sensor 10 for acquiring the respiratory flow, respiration rate, one or more gas component or gas concentration signal can be placed on a skin below a nose but above the subject's upper lip or mouth, where it is glued on with a sticker 12 located on the bottom of the sensor 10.

The sensor 10 may connect to the housing 5 of the monitoring system 1 through a cable 13, which is preferably deformable and retain a shape. So the cable 13 can have rigid, but flexible mechanical properties, so that it can be shaped by forcing or bending it to fit different type of head geometries of different subjects. It is advantageous if the cable 13 also retain the reshaped shape in the normal use, such as the subject sleeps and the subject's head presses the ear handle, which remains between the head and the base on which the subject lies on. Furthermore, the cable 13 could also imitate the head or bend to make it unnoticeable for the subject. The cable 13 can also be a relatively thin flat or round band, preferably comprising plastic or similar non-irritating, clinically approved material.

The cable 13 also comprising electrical wires preferably inside the band that are used to transfer electrical signals between the sensor 10 and the housing 5 of the monitoring system 1. The cable 13 comprises a connector 14, preferably a male type connector at the sensor end of the band (not shown in figures) that mechanically locks-on to the corresponding counterpart connector 15 in the sensor 10, which is preferably a female type connector. Connectors 14 and 15 also connect electrically the sensor 10 through the electrical contacts and wires into the housing 5 of the monitoring system 1. Thus the sensor 10, preferably disposable but it may also be reusable, is easy to unlock from the cable 13 with one press on the latch pin (not shown in Figures), which may be similar to for example standard Ethernet connector latch pin. The electrical operating power of the power supply 7 and possible control signals for the sensor 10 are transferred from the housing 5, whereas electrical signals indicative of the condition of the respiratory function are transferred from the sensor 10 to the housing 5.

The other end of the cable13 connects in Figure 1 embodiment tightly to the housing 5 of monitoring system 1, but naturally can also be detachable. The housing 5 and the cable 13 are preferably reusable, thus they are designed and made of materials that are easy to clean.

The housing 5 may also be operatively connectable to another sensor 20 for acquiring a signal indicative of a condition of the respiratory function such as an absorbance of a blood which is indicative for example of an oxygen saturation and/or pulse rate. In Figure 1 embodiment there is a cable 22 connecting the housing and the sensor 20. In this case the housing is equipped with a connector 16, which is preferably the same female type connector as the connector 15 in the sensor 10, or similar to that. An end of the cable 22 is equipped with a connector 17, which is preferably the same male type connector as the connector 14 in the cable 13, or similar. The connector 17 connects to its counterpart connector 16, both mechanically and electrically, similarly as the cable 13 connects to the sensor 10. Thus the sensor 20, which may be disposable or reusable, is easy to unlock from the housing 5 with one press on the latch pin (not shown in Figures), which may be similar to for example standard Ethernet connector latch pin. The electrical operating power and possible control signals for the sensor 20 are transferred from the housing 5, whereas the acquired electrical signals from the sensor 20 are transferred to the housing 5 for further processing.

The sensor 20 shown in Figure 1, which is a preferably a disposable pulse oximeter sensor placed on the ear lope, allows a non-invasive method of monitoring the oxygenation of the subject's hemoglobin and can be used to acquire a signal indicative of oxygen saturation in blood and pulse rate. The sensor 20 is placed on a thin part of the subject's anatomy, usually a fingertip or the ear lope, or in the case of a neonate, across a foot. The sensor 20 comprises an emitting unit 25 to emit a radiation through a tissue of the subject and a detector unit 26 to receive the radiation penetrated through a tissue and emitted by the emitting unit 25. The detector unit 26 creates a signal indicative of an absorbance of the blood and which signal is provided to the housing 5 along the cable 22. The light emitting unit 25 is able to emit at least two different wavelengths of the radiation. Typically the emitting unit 25 comprises one radiation emitter 27 for a red wavelength and one radiation emitter 28 for an infrared wavelength. The detector unit 26 may comprise one or more detectors 29, 30 to receive the radiation emitted by the radiation emitters 27, 28. Changing absorbance of each of the two wavelengths is measured, allowing determination of the absorbances due to the pulsing arteria blood alone, excluding venous blood, skin, bone, muscle, fat. Based upon the ratio of changing absorbance of the red and infrared wavelengths caused by the difference in color between oxygen-bound (bright red) and oxygen unbound (dark red or blue, in severe cases) blood hemoglobin, a measure of oxygenation, the per cent of hemoglobin molecules bound with oxygen molecules, can be made.

The monitoring system 1 may also comprise a third sensor 40, which is indicative of a condition other than the respiration function and preferably a disposable temperature measurement used to measure the body temperature of the subject. The housing 5 comprises a connector 41, which is preferably a similar female type connector as the connector 15 and 16. The sensor 40 is connected by means of a cable 42 and its connector 43 at the end of this cable 42, which is preferably a similar male type connector as the connectors 14 and 17. The connector 43 connects to its counterpart connector 41, both mechanically and electrically. Thus the sensor 40 is easy to unlock from the housing 5 with one press on the latch pin (not shown in Figures), which may be similar to for example standard Ethernet connector latch pin. The electrical operating power and possible control signals for the sensor 40 are transferred from the housing 5, whereas the acquired electrical signals from the sensor 40 are transferred to the housing 5 for further processing.

The housing 5 is able to communicate with a host unit 50. All the processed or unprocessed data acquired from the sensors 10, 20 and 40 is sent to the host 50 preferably through a communication link 51, which is preferably wireless. The host unit can further process the signal managed by the electronics 6 of the housing 5 and also display the processed signal by means of a display unit 52.

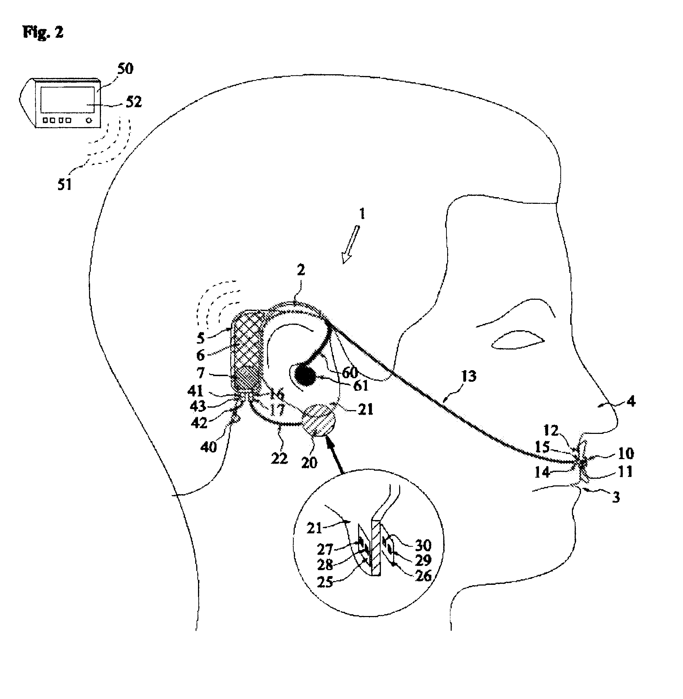

Figure 2 is mainly identical with Figure 1, but it further shows an ear handle 60 coupling the housing 5 to the subject's ear 2 .The embodiment shown in Figure 1 is coupled to the ear by means of the cable 13 or a separate sticker (not shown in Figure 1), but in some cases an improved coupling may be necessary. The ear handle 60 shown in Figure 2 may be coupled to the cable 13 or it may be coupled to the housing 5 so that it alone or together with the cable 13 encircles at least partly the ear 2. Also the ear handle 60 may comprise an auditory canal element 61 insertable into the auditory canal for better fastening the housing 5. Also the auditory canal element 61 can be an hearing aid insertable into the auditory canal. Further the auditory canal element 61 can be a sensor for measuring the signal indicative of the condition of the respiratory function such as oxygen saturation and pulse rate replacing the subject's ear lope 21 as a place for sensor 20.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects of the present invention are defined in the following numbered clauses:
1. A wearable monitoring system to collect medical information from a subject comprising:
   a housing having electronics for managing a signal and which at least one housing is operatively connectable to at least one sensor for transmitting to said housing the signal acquired by said at least one sensor;
   wherein said electronics of said housing is for managing the signal which is indicative of a condition of a respiration function and which housing is installable to the subject's ear or its vicinity.
2. The wearable monitoring system according to clause 1 further comprising a cable operatively connectable to said at least one sensor for transmitting to said housing the signal acquired by said at least one sensor.
3. The wearable monitoring system according to clause 1 or clause 2, wherein said housing also comprising a power supply is configured to create an electrical operating power to transmit the energy from said housing to said sensor.
4. The wearable monitoring system according to any one of the preceding clauses, wherein the signal indicative of the condition of the respiratory function is at least one of a respiration flow and respiration rate.
5. The wearable monitoring system according to any one of the preceding clauses, wherein the signal indicative of the condition of the respiratory function is at least one of oxygen saturation and pulse rate.
6. The wearable monitoring system according to any one of the preceding clauses, wherein the signal indicative of the condition of the respiratory function is one or more gas component or a concentration of said gas component.
7. The wearable monitoring system according to any one of the preceding clauses, wherein said sensor comprising a respiration detector is configured to acquire the signal indicative of the respiration flow and respiration rate.
8. The wearable monitoring system according to any one of the preceding clauses, wherein said sensor comprising an emitting unit to emit a radiation through a tissue and a detector unit receiving the radiation emitted by the emitting unit is configured based on the received radiation to create a signal indicative of an absorbance of a blood and to provide the signal to said housing.
9. The wearable monitoring system according to clause 8, wherein said emitting unit is configured to emit at least two different wavelengths of the radiation and said detector unit is configured to receive said at least two different wavelengths and said housing is configured to determine one of oxygen saturation and pulse rate based on the signal created by said detector unit.
10. The wearable monitoring system according to any one of the preceding clauses, wherein said sensor comprising a respiration detector is configured to acquire a signal indicative of one or more gas component or a concentration of a gas component.
11. The wearable monitoring system according to any one of the preceding clauses, further comprising a sensor, which is configured to measure a body temperature of the subject.
12. The wearable monitoring system according to any one of the preceding clauses, wherein said at least one sensor is disposable.
13. The wearable monitoring system according to any one of the preceding clauses, wherein said housing is reusable.
14. The wearable monitoring system according to clause 2, wherein said cable is deformable and retain a shape.
15. The wearable monitoring system according to any one of the preceding clauses, wherein said housing is configured to communicate with a host unit for further processing of the signal managed by said electronics.
16. A wearable monitoring system to collect medical information from a subject comprising:
   a housing having electronics for managing a signal;
   a cable operatively connectable to at least one sensor for transmitting to said housing the signal acquired by said at least one sensor;
   wherein said electronics of said housing is for managing the signal acquired along said cable from said at least one sensor and which signal is indicative of a condition of a respiration function and which housing is installable behind an ear of the subject.
17. The wearable monitoring system according to clause 16, wherein said housing and said cable are connected together and are reusable.
18. The wearable monitoring system according to clause 16 or clause 17, wherein said housing is configured to communicate with a host unit for further processing of the signal managed by said electrical components and to display said processed signal.
19. A wearable monitoring system to collect medical information from a subject comprising:
   at least one sensor for acquiring a signal indicative of a condition of a respiration function; and
   a cable for transmitting the signal acquired by said at least one sensor; and
   a housing having electronics for managing the signal received along said cable from said at least one sensor and which housing is installable behind an ear of the subject.
20. The wearable monitoring system according to clause 19, wherein the signal indicative of the condition of the respiratory function is at least one of a respiration flow, respiration rate, oxygen saturation, pulse rate, one or more gas component and a concentration of said gas component.

## Claims

1. A wearable monitoring system to collect medical information from a subject comprising:
a housing (5) having electronics (6) for managing a signal and which at least one housing is operatively connectable to at least one sensor (10,20) for transmitting to said housing (5) the signal acquired by said at least one sensor,
**characterized in that** said electronics (6) of said housing (5) is for managing the signal which is indicative of a condition of a respiration function and which housing is installable to the subject's ear (2) or its vicinity.

2. The wearable monitoring system according to claim 1, further comprising a cable (13) operatively connectable to said at least one sensor (10,20) for transmitting to said housing (5) the signal acquired by said at least one sensor.

3. The wearable monitoring system according to claim 1 or 2, **characterized in that** said housing (5) also comprising a power supply (7) is configured to create an electrical operating power to transmit the energy from said housing to said sensor (10,20).

4. The wearable monitoring system according to any one of the preceding claims, **characterized in that** the signal indicative of the condition of the respiratory function is at least one of a respiration flow and respiration rate.

5. The wearable monitoring system according to any one of the preceding claims, **characterized in that** the signal indicative of the condition of the respiratory function is at least one of oxygen saturation and pulse rate.

6. The wearable monitoring system according to any one of the preceding claims, **characterized in that** the signal indicative of the condition of the respiratory function is one or more gas component or a concentration of said gas component.

7. The wearable monitoring system according to any one of the preceding claims, **characterized in that** said sensor (10) comprising a respiration detector (11) is configured to acquire the signal indicative of the respiration flow and respiration rate.

8. The wearable monitoring system according to any one of the preceding claims, **characterized in that** said sensor (20) comprising an emitting unit (25) to emit a radiation through a tissue and a detector unit (26) receiving the radiation emitted by the emitting unit is configured based on the received radiation to create a signal indicative of an absorbance of a blood and to provide the signal to said housing (5).

9. The wearable monitoring system according to claim 8, **characterized in that** said emitting unit (25) is configured to emit at least two different wavelengths of the radiation and said detector unit (26) is configured to receive said at least two different wavelengths and said housing (5) is configured to determine one of oxygen saturation and pulse rate based on the signal created by said detector unit.

10. The wearable monitoring system according to any one of the preceding claims, **characterized in that** said sensor (10) comprising a respiration detector (11) is configured to acquire a signal indicative of one or more gas component or a concentration of a gas component.

11. The wearable monitoring system according to any one of the preceding claims, further comprising a sensor (40), which is configured to measure a body temperature of the subject.

12. The wearable monitoring system according to any one of the preceding claims, **characterized in that** said at least one sensor (10,20) is disposable.

13. The wearable monitoring system according to any one of the preceding claims, **characterized in that** said housing (5) is reusable.

14. The wearable monitoring system according to any one of the preceding claims, **characterized in that** said cable (13) is deformable and retain a shape.

15. The wearable monitoring system according to any one of the preceding claims, wherein said housing is configured to communicate with a host unit (50) for further processing of the signal managed by said electronics.
